# EUROPEAN PATENT SPECIFICATION

(11) **EP 1 945 795 B1**
(45) Date of publication and mention of the grant of the patent: **10.08.2016**
(21) Application number: 06825268.3
(22) Date of filing: 27.09.2006
(51) Int. Cl.: C12Q 1/02, C12Q 1/68, G01N 33/53

(54) **METHODS AND CONSTRUCTS FOR ANALYZING BIOLOGICAL ACTIVITIES OF BIOLOGICAL SPECIMENS AND DETERMINING STATES OF ORGANISM**
VERFAHREN UND KONSTRUKTE ZUR ANALYSE BIOLOGISCHER AKTIVITÄTEN BIOLOGISCHER PROBEN UND ZUR BESTIMMUNG VON ZUSTÄNDEN EINES ORGANISMUS
PROCÉDÉS ET CONSTRUCTIONS PERMETTANT D'ANALYSER DES ACTIVITÉS BIOLOGIQUES DE SPECIMENS BIOLOGIQUES ET DÉTERMINER DES ÉTATS D'UN ORGANISME

(30) Priority: 28.09.2005 US 721860 P
(43) Date of publication of application: 23.07.2008
(73) Proprietor: Attagene, Inc., North Carolina 27709 (US)
(72) Inventor: ROMANOV, Sergei, Chapel Hill, NC 27517 (US); MAKAROV, Sergei, Chapel Hill, NC 27516 (US)
(74) Representative: Schüssler, Andrea
(86) International application number: PCT/US2006/038175
(87) International publication number: WO 2007/038756

(56) References cited:
- WO-A2-02/099117
- WO-A2-03/076932
- GB-A- 2 355 791
- US-A1- 2002 177 218
- US-A1- 2003 108 877
- US-A1- 2006 160 108
- EMILIEN G. ET AL.: 'Impact of Genomics on Drug Discovery and Clinical Medicine' Q. J. MED. vol. 93, no. 7, July 2000, pages 391 - 423, XP000941207

## Description

### 2. FIELD OF THE INVENTION

This application relates to methods of analyzing transcriptional activities of transcription factors and cis-regulatory elements in a cell, for example, to determine a biological activity in a sample applied to the cell.

### 3. BACKGROUND

In multicellular organisms, cells communicate by releasing myriads of signals, such as neuromediators, hormones, growth factors, cytokines, etc. These mediators carry specific instructions as to how particular cell types, organs, and tissues, should alter their behavior.

States of the host (e.g., health vs. disease) can be analyzed by assessing the spectra of biological activities in its biological fluids in regard to their actions on different cell types and tissues.

Several approaches for analyzing the content of biological fluids and other samples are being developed, such as proteomics that evaluates concentration profiles of proteins in biological specimens, e.g., by using antibody arrays, and metabolomics, wherein profiles of biological mediators are evaluated according to their weights and molecular structures, e.g., by using chromatography, mass-spectrometry, etc. However, analyzing the physical-chemical properties of individual constituents provides little information about the biological activities of evaluated samples.

Biological activity can be directly assessed by using various cell-based assays, where analyzed samples are contacted with tester cells in culture and phenotypical changes (e.g., apoptosis, proliferation, differentiation) of tester cells are evaluated. However, as there are many distinct molecules that can elicit same phenotypical changes in tester cells, those assays can be used for detecting certain (e.g., pro-apoptotic, mitogenic, etc.) activities but are poorly suited for analyzing the complex spectra of biological activities in the samples.

US2003/108877 discloses cell lines stably transfected with a reporter gene under the transcriptional control of a particular transcription factor or family thereof endogenous to the host cell.

A cell-based assay recently proposed characterizes evaluated biological samples according to the alterations in gene expression occurring in tester cells in response to contact with the sample. In this approach, the response of the tester cells is analyzed by assessing the profile of gene expression (transcriptome) in these cells, e.g., by hybridizing cellular RNA to detection array (USPTO publication No. 2005/0181354 A1).

Such an approach, however approach has several shortcomings. One is that analyzing the transcription response requires the analysis of expression of tens of thousands genes. Another challenge is how to interpret the large amounts of data produced by microarrays. To find the characteristic patterns in expression of thousands of genes, algorithms have been developed that allow identifying the clusters of genes regulated in a similar fashion (see, e.g., Hughes et al., 2000, J. Mol. Biol. 296:1205-1214) but this problem still requires further integration of higher-order statistical analyses and data management. Thus, such assays are laborious, expensive, and their results are difficult to interpret in regard of biological activities of analyzed samples.

### 4. DESCRIPTION OF INVENTION.

An alternative to describing cells at the level of gene expression (i.e., transcriptiomics) is to investigate the molecular changes occurring at a signal transduction level. In response to cell stimulation, cell activates the signal transduction pathways that result in alteration of gene expression. At the apex of most signal transduction pathways lay inducible transcription factors (TFs), the proteins that bind specific DNA sequences within the promoter regions of genes, thereby initiating or repressing transcription. Activity of TFs is regulated at many levels, such as post-translational modification (e.g., phosphorylation or acetylation), degradation, nuclear translocation, DNA binding, and/or by interactions with other proteins, including the basal transcriptional machinery, co-activators or co-repressors, and other TFs. These different levels of regulation allow gene expression to be tightly controlled. Through different combinations of these regulatory mechanisms, eukaryotes are able to elicit a myriad of gene expression patterns.

An approximate 2,000 different TFs that comprise several hundreds of distinct TF families exist in the human genome. These TF families orchestrate the expression of tens of thousands genes. As the complexity of cell regulation is dramatically reduced at the TFs level, analyzing the molecular changes at the TFs level enables much simpler and comprehensive interpretation of biological activities of tested compounds.

The present invention provides methods whereby the spectra of biological activities of biological specimens are analyzed by assessing their effects on the signal provided methods of analyzing the activities of transcription factors (TFs), as well as the transcriptional activities of cis-regulatory response elements (cisREs) that are regulated by these TFs. Advantages of this invention include, for example, that the functional states of organism are characterized by analyzing the biological activities of biological samples derived from the organism. This obviates the necessity of introducing reporter systems into evaluated host and thus provides the opportunity of a non-invasive assessment. Furthermore, the invention affords the assessment of collections of archived materials, e.g., serum, tissues, etc. The present invention provides methods of analyzing the transcriptional activities of TFs and cisREs, methods of deriving information about the biological activities of various biological specimens and methods of identifying selective markers of disease, evaluating drug candidates, discovering the targets for therapeutic treatments, among many other biomedical applications.

Specifically, the present invention provides a method of determining biological activities of a biological sample which has been obtained from an organism, comprising:
(i) contacting the biological sample obtained from the organism with a cultured biosensor cell comprising a library of transcription reporter constructs for assessing transcriptional activities of multiple transcription factors (TFs) in the cultured biosensor cell,
   wherein each transcription reporter construct in the library comprises a cis-regulatory element responsive to a TF and a reporter sequence,
   wherein the reporter sequence in each transcription reporter construct in the library comprises a processing tag that is located in a position within the reporter sequence that differs from the positions of the processing tag in the reporter sequence of the other reporter constructs in the library, and
   wherein the reporter sequences of the transcription reporter constructs, outside of the positions of the processing tags, are identical;
(ii) assessing relative levels of transcripts of the transcription reporter constructs in the library within the cultured biosensor cell to obtain a profile of activities of TFs regulating transcriptional activities of the reporter constructs in the library in the cultured biosensor cell;
(iii) comparing the profile of activities of TFs from the cultured biosensor cell contacted with the biological sample to a profile of activities of TFs in a cultured biosensor cell that was not contacted with the biological sample; and
(iv) generating a differential profile comprising differences between the profile of TF activities from the cultured biosensor cell contacted with the biological sample and the profile of activities of TFs in the cultured biosensor cell not contacted with the biological sample, wherein the differential profile represents the biological activities of the biological sample obtained from the organism.

According to one embodiment of the invention, the method of the invention further comprising:
(v) contacting the biological sample obtained from the organism with a second cultured biosensor cell for a period of time different than the cell in step (i) was contacted, wherein the second cell comprises the library of transcription reporter constructs;
(vi) assessing relative levels of transcripts of the transcription reporter constructs in the library within each of the two cultured biosensor cells contacted in steps (i) and (v) to obtain a profile of activities of TFs regulating transcription of the transcription reporter constructs in the library in each of the two cultured biosensor cells;
(vii) comparing the profile of activities of the TFs in each of two cultured biosensor cells contacted with the biological sample with a profile of activities of the TFs in a cultured biosensor cell that was not contacted with the biological sample, and
(viii) generating a differential profile comprising differences in the profiles of the two biosensor cells contacted with the biological sample to a profile of activity of the TFs in cultured biosensor cell not contacted with the biological sample that represents the biological activities of the biological sample obtained from the organism.

Finally, the present invention provides a method of identifying markers of a disease in a sample which has been obtained from an organism, comprising:
(i) determining, in accordance with the method of claim 1, biological activities of a biological sample obtained from the organism having the disease;
(ii) determining, in accordance with the method of claim 1, biological activities of a biological sample obtained from an organism not having the disease;
(iii) comparing the biological activities of the biological sample from the organism having the disease to the biological activities of the biological sample from the organism not having the disease; and
(iv) generating a profile of differences between the compared biological activities wherein the generated profile identifies markers of the disease.

The biological activity of analyzed sample is defined through the ability of the sample to induce changes in activities of signal transduction pathways in tester cell system hereafter called biosensor. The alterations in the activities of the signals transduction pathways are evaluated by assessing the profiles of activities of TFs and/or cis-REs in these biosensors.

The invention is based, in part, on the premises that:
(i) the state of biological system can be characterized by analyzing the biological activities of its constituents (e.g., biological fluids, tissue extracts, or other specimens);
(ii) the biological activities in analyzed sample can be assessed by contacting the sample with a tester cell system (hereafter termed biosensor) and by determining alterations in signal transduction within the biosensor;
(iii) the alterations in signal transduction can be comprehensively described by assessing profiles of activities of multiple transcription factors (TFs) or profiles of activities of reporter constructs that contain cis-response elements (cisREs) controlled by those TFs;
(iv) sufficient resolution of biosensors can be achieved to distinguish different states of analyzed biological system.

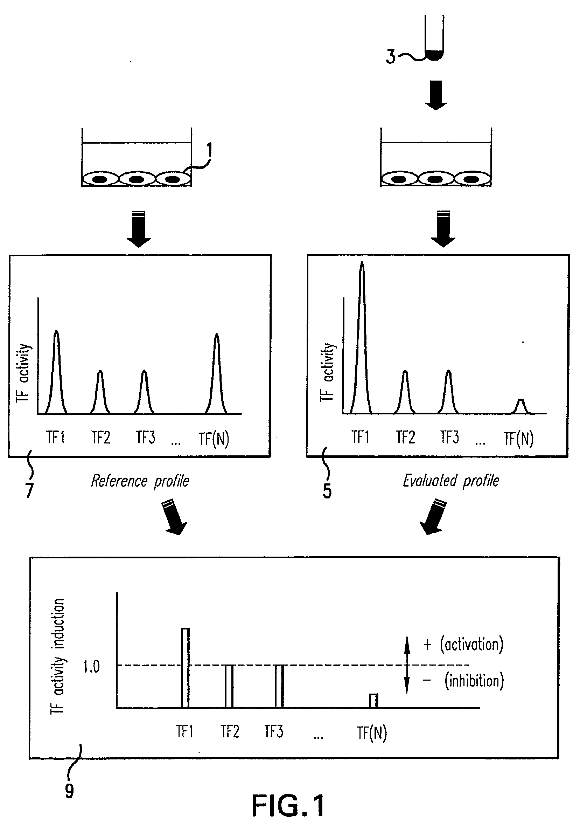

Fig. 1 depicts one ramification of the invention. In this example, biosensor 1 is a homogenous population of one cell type that is maintained in culture under standard growth conditions. The biosensor is contacted with analyzed sample 3 by adding the analyzed sample to the growth medium for a defined period of time. At the end of incubation, a determination of the profile of activities of TFs in the biosensor is made and thus determines the evaluated TF activity profile 5. A determination of the reference profile of activities of the TFs 7 in the biosensor that was not contacted with the evaluated sample can be made. By comparing the profiles of TF activities (evaluated vs. reference), one determines the changes in activities of individual TFs occurring in response to the analyzed sample. The resulting profile of alterations of TF activities 9 represents a molecular signature of the biological activity of the evaluated sample 3.

Activities of TFs within the biosensor can be determined by using different approaches.

A TF activity may be assessed by measuring the binding activity of the TF to a DNA probe comprising a TF-binding sequence. This can be done by assaying cellular extracts in any available DNA binding assay, e.g., a gel-shift assay (also know as electromobility shift assay, or EMSA), an ELISA-based DNA binding assay, etc.

According to the invention, the transcriptional activity of TFs, i.e., the ability to activate the expression of target genes, are evaluated. To do so, biosensor cells are supplied with a library of reporter constructs enabling the assessment of multiple TFs and cisREs, and the activities of evaluated TFs are assessed by analyzing the activities of corresponding reporter constructs. Many reporter constructs are available for this purpose, e.g., luciferase, CAT, GFP reporters, etc. The activities of multiple TFs can be assessed in parallel by using libraries of reporter RNA constructs (U.S. patent publication No. 2006/0160108).

By comparing the signature of evaluated sample with a database comprising the molecular signatures of other samples, one can relate the evaluated sample to other samples. To this purpose, mathematical algorithms exist that can quantitatively compare the TF activity profiles, e.g., correlation analysis. Various parametric and non-parametric metrics are available for this purpose, e.g., Euclidian distance, Pearson's correlation coefficient, rank order correlation, etc.

The resolution can be defined as the capability to distinguish between different biological activities. For example, many distinct molecules exist, e.g., interleukin-1 (IL-1), tumor necrosis factor alpha (TNFa), bacterial lipopolysaccharide (LPS), that can induce the activation of the transcription factor NF-kB. Thus having observing the activation of NF-kB per se cannot distinguish between those mediators. However, in many circumstances, it is important to have a capability to distinguish those molecules. For example, the presence of TNFa and IL-lb in circulation may indicate chronic inflammatory disease or endotoxemia, while the presence of LPS is indicative of endotoxemia.

There are several distinct approaches whereby the resolution of the assay can be further optimized.

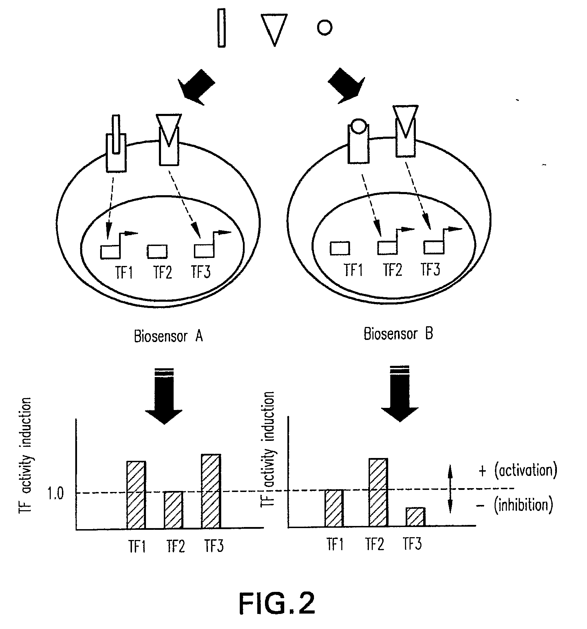

In one approach, the sample is analyzed by using a panel of biosensors that represent different cell types. That is, the molecular signatures of the sample are assessed by contacting the sample with biosensors representing epithelial cells, immune cells, fibroblasts, neural cells, etc., and the profiles of TF activities in those cells are assessed. It is anticipated that the molecular signatures will be different in different cell types. For example, LPS will activate NF-kB in the cells that express an LPS receptor (e.g., TLR-4), but not in the cells that lack this receptor. Similarly, the presence of TNFa in the sample will activate NF-kB in cells that express TNFa receptors, but not within cells lacking these receptors, etc. Therefore, in order to distinguish between two different biological activities, one should expand the panel of biosensors by including different cell types until the desirable resolution is achieved (Figure 2).

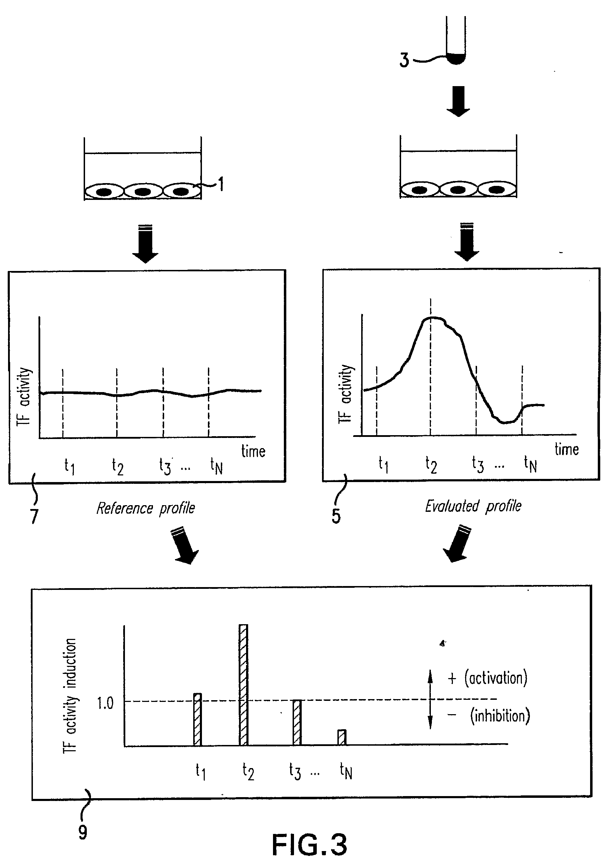

In another approach, differential biological activities can be distinguished by analyzing temporal patterns of TF activity profiles. For example, TNFa and IL-1b induce a very rapid activation of NF-kB that reaches a peak within minutes and then subsides. Two to three hours later, the second wave of NF-kB activation occurs. By contrast, platelet-derived growth factor (PDGF) causes a slow activation of NF-kB that reaches a peak within hours of stimulation (Romashkova and Makarov, 1999). Therefore, in order to distinguish between two different biological activities, one can compare the profiles of TF activities at various time points after the contact with the samples (Figure 3).

In yet another approach, the resolution can be increased by increasing the number of TFs that are assessed in the assay. For example, LPS activates both NF-kB and interferon-response elements, while TNFa activates only NF-kB. Thus, by assessing the activities of NF-kB and IFNg-responsive elements, one can distinguish between LPS and TNFa in the analyzed sample. Therefore, in order to distinguish between two different biological activities, one should expand the number of evaluated TFs until desirable resolution is achieved.

In yet another approach, biosensor is contacted with analyzed sample in the presence of a response-modifying agent. For example, inflammation often results in the release into circulation of anti-inflammatory molecules, such as IL-1 receptor antagonist, corticosteroids, soluble TNFa receptors, etc. These molecules may not necessarily induce alterations in signal transduction within biosensor, but may selectively prevent the activation of TFs by cytokines. For example, if evaluated sample inhibits activation of NF-kB in response to cytokine IL-1, but not in response to TNFa, this may indicate the presence of selective inhibitors of IL-1. In contrast, a selective suppression of TNFa-inducible NF-kB activation by the evaluated sample will indicate the presence of selective inhibitors of TNFa. Thus, by combining the evaluated sample with response-modifying agents, one can increase the resolution of assay. Various agents can be used as response-modifying agents, such as cytokines and mixture of cytokines, growth factors, low-molecular weight compounds, radiation, etc. Also, the TF activity profile in biosensors can be altered by using various expression constructs, e.g., by expressing cDNAs encoding various genes, as well as dominant-negative and constitutively active variants of those genes. Furthermore, many different ways that alter gene expression within biosensors can be used, including antisense molecules, small interfering RNAs, etc. Therefore, to distinguish between two different biological activities, one evaluates the biological activities of samples in the presence of various response-modifying agents, until desirable resolution is achieved.

Various biological systems can be used as biosensors. For example, biosensor can be a homogenous cell culture comprising one cell type. The biosensor can also comprise a mixed population of different cell types. Biosensor can also comprise a tissue or an organ culture, e.g., brain slice culture, liver slice culture, skin flap, etc. Also, a cell population, organ, or tissue can be engrafted into animals to serve as an in situ biosensor. For example, the engrafted tissue, organ, or cell population can be supplied with a library of reporter constructs, and the monitoring of reporter constructs' expression will provide information about biological activities of fluids and tissues contacting the engrafts. Whole organs and tissues of live animals can also be used as biosensors. For example, an isolated liver of live animal can be perfused with analyzed sample followed by assessment of TF activity profiles within the liver, or skin of animal can be contacted with analyzed sample, followed by assessment of TF activity profiles within the skin.

Various biological samples can be analyzed by this invention, e.g., biological fluids, including saliva, blood, serum, cerebrospinal fluid, synovial fluid, urine, semen, breast milk, bile, tears, feces extracts, etc., as well as extracts, concentrates, components, or fractionates thereof. One can also analyze biological activities of cellular and tissue extracts, conditioned cell culture medium, etc. Furthermore, various cells can also be considered as biological samples. In this regard, the biological activity can be defined as the alteration in signal transduction that occurs upon cell-cell contacts of biosensors with the samples, i.e., live or fixed (e.g., glutaraldehyde-fixed, formalin-fixed) cells, or cell membranes.

The present invention provides means to characterize functional state of a biological system thru assessment of biological activities of its constituents. Variety of biological systems can be characterized in this way, including cell cultures, mixed population of cells, tissue and organ cultures, engrafted cells and tissues, organs and tissues of live animals, or whole live animals. To characterize the biological system, one collects biological samples from this system (cell supernatants, tissue extracts, bodily fluids, etc.), contacts these samples with biosensors, and determines alterations in signal transduction in the biosensors (i.e., alterations in profiles of TFs activities).

The invention is further useful for the identification of markers of perturbed functional states of various biological systems. For example, perturbed state of an animal can be a disease. To determine markers of the disease, one assesses biological activities of one or several biological samples from the diseased animal and the biological activities in corresponding samples from undisturbed (healthy) animal, and, by comparing biological activities in these samples, one identifies markers of the disease. For example, if serum of animal with a certain disease induces activation of certain TFs in biosensors, while serum of healthy animals does not, then activation of these TF provides a marker of the disease. The differentially inhibited TFs also provide the markers of the disease.

Furthermore, one can assess the intensity of perturbation (e.g., the severity of a disease) by quantitatively evaluating the intensity of the marker of said perturbation.

Different kinds of perturbations can be assessed, including a disease, a pre-disease state, aging, different treatments that alter the functional state of biological system, e.g., stress, diet, therapeutic treatment, administration of chemical compounds, toxins, pathogens, etc.

The invention establishes method of identifying markers that distinguish different perturbed functional states of a biological system. To do so, one determines the biological activities of one or multiple biological samples derived from the biological system in one perturbed state and in another perturbed state of organism, and, by comparing those biological activities, identifies the markers that distinguish those perturbed states.

The invention thus may be used in a method of diagnostics of disease and pre-disease states of an organism. To do so, one determines the biological activities in one or multiple biological samples derived from the evaluated organism and compares these biological activities with database of markers of diseases and pre-disease states. Various diseases can be diagnosed in this way, including chronic inflammatory diseases (e.g., arthritis, lupus, etc.), metabolic diseases (such as diabetes), various cancers, neurodegenerative diseases (e.g., Alzheimer disease, Parkinson disease, etc.), psychosomatic disease, various infections (e.g., bacterial and viral infections), hereditary diseases (e.g., premature aging), pre-infarction state, pre-diabetic state, etc.

The invention may be used in a method of identification of putative therapeutic targets and drug candidates for various diseases and pre-diseased states. To do so, one identifies markers of said disease and pre-disease states. As discussed above ([0028]), those markers represent TFs that are upregulated or downregulated in biosensors by contact with samples derived from diseased organisms. Therefore, these markers represent putative therapeutic targets. For example, if a marker of the disease is upregulated NF-kB activity, then inhibitors ofNF-kB,-or inhibitors of the upstream signal transduction cascades controlling NF-kBF,-represent a putative treatment for the disease. Vice versa, if a certain TF is inhibited, activators of this TF may represent a putative treatment.

Akin to that, present invention may be used in a method of evaluating the efficacy of drug candidates and other treatments for a disease.

### 5. EXAMPLES

The working examples below demonstrate the successful assessment of biological activities of sera derived from healthy and diabetic animals, using different types of reporter cells to determine non-redundant set of biological activities present in the serum of diabetic animals, and how information can be assembled to identify a disease.

### 5.1. Example 1

Materials and general procedures used in the examples that follow are described below.

**Animals.** Manipulations with experimental rats were performed in certified animal facility and according to the approved animal protocol.

**Cells.** Human hepatocellular carcinoma, HepG2, embryonic kidney epithelial, HEK293 and rat insulinoma, U7, cell lines were maintained on DME media (Invitrogen, Carlsbad, CA, USA) supplemented with 10% FBS (HyClone, Logan, UT, USA) and further supplemented with antibiotics. Streptozotocin was purchased from Sigma (Sigma-Aldrich, St. Louis, MO, USA).

**Plasmid DNA manipulations.** Manipulations with plasmid DNAs were performed using standard molecular biology techniques known in the art, as described, for example, in Sambrook and Russell, Molecular Closing: A Laboratory Manual, 3rd Ed. (Cold Spring Harbor Laboratory Press, Cold Spring Harbor, N.Y., 2001) and Current Protocols in Molecular Biology (Ausubel et al., eds., John Wiley & Sons, 1994-1998, Current Protocols, 1987-1994, as supplemented through July 2005 (Supplement 71)).

**Transfections.** For transfections, the cells were plated at a subconfluent density (5x10⁵/well) in wells of a 12 well plate. Eighteen hours later, cells were transfected plasmid DNA at a ratio of 1.5 µl /0.5 µg of total plasmid DNA for each transfection, according to the manufacturer's protocol. The day after transfection, the medium was replaced with one ml of fresh growth medium.

**Isolation of cellular RNA.** Total cellular RNA was isolated by using TRIZOL reagent (Invitrogen, Carlsbad, CA, USA) according to the manufacturer's protocol and re-dissolved in water. Routinely, .5 ml of the TRIZOL reagent was used to extract RNA from the confluent monolayer of cells in a well of a 12-well plate.

**RT-PCR.** Samples of total RNA were treated with DNAse I (Ambion, Austin, TX USA) according to manufacturer's instructions. Residual DNAse was heat inactivated at 70° C for 15 min. The DNAse-treated RNA was reversely transcribed by using oligo-dT polynucleotides and Mo-MLV reverse transcriptase (Invitrogen, Carlsbad, CA, USA) according to the manufacturer's instructions. One tenth of the reversely transcribed RNA was amplified in a PCR reaction, by using TAQ DNA polymerase (Invitrogen Carlsbad, CA, USA) and the following reporter sequence-specific primers: (forward primer: 1: 5'-AAATACGAGATCCACCGAGACTCC-3' **(SEQ ID NO: 1)** and reverse primer 2: 5'-GCAGGAACAGCGCCGATACAAT-3' **(SEQ ID NO: 2)).** PCR conditions used were similar, or identical, to those described in U.S. patent publication No. 2006/0160108. PCR reactions were performed on a ABI 9700 GENEAMP thermo-cycler.

**Labeling of PCR products.** One tenth of each completed PCR reaction was diluted with a fresh PCR reaction mixture containing 6-Carboxyfluorescein (6-FAM) 5'-labeled reporter polynucleotide-specific primer (primer 2: 5'-GCAGGAACAGCGCCGATACAAT-3') and then incubated at 95°C for 2 min, at 68°C for 20 sec and at 72°C for 10 min.

**Endonuclease restrictions.** Hpa I restriction endonuclease (New England Biolabs, Ipswich, MA, USA) was directly added to the labeled PCR products at concentration of 5 U/reaction. The samples were digested for 2 hrs and purified using Qiaquick PCR purification columns (Qiagen, Hilden, Germany) according to the manufacturer's protocol.

**Capillary electrophoresis.** Serial dilutions of each Hpa I digested sample were analyzed by capillary electrophoresis using ABI PRIZM 3100 genetic analyzer (Applied Biosystems, Foster City, CA, USA). A set of X-rhodamine-labeled MAPMARKER1000 molecular weight standards (Murfreesboro, TN USA) was run in parallel to the analyzed samples as a molecular weight reference.

### 5.2. Example 2

The working examples below demonstrate the successful assessment of biological activities of sera derived from healthy and diabetic animals.

Experimental type I diabetes mellitus was induced in rats by using Streptozotocin (STZ), a *N*-nitroso derivative of D-glucosamine. Streptozotocin causes rapid necrosis in pancreatic ß-cells of islets of Langerhans (Okamoto, 1985, Bioessays 2:15-21). STZ has been widely used to provoke insulin-dependent diabetes conditions in various laboratory animals, and animals treated with STZ are recognized in the art as a model of diabetes mellitus (Like and Rossini, 1976, Science 193:415-417).

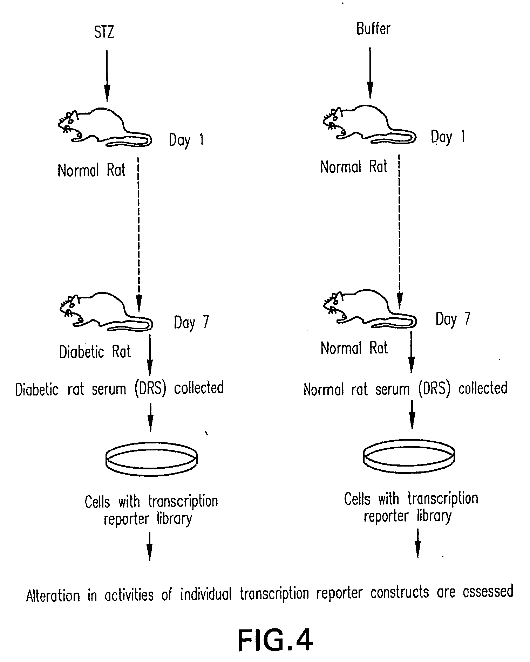

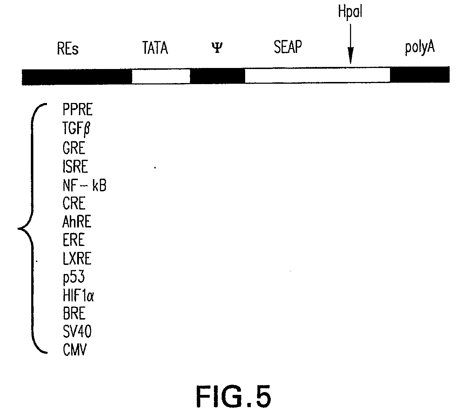

The design of the approach to compare sera from diabetic and healthy rats is illustrated in **Figure 4****.** Sprague-Dawley male rats aged 10-12 weeks were randomly allocated in two groups. Rats from one group received a single intra-peritonial injection of 70 mg/kg STZ (Sigma-Aldrich, St. Louis, MO, USA) dissolved in freshly prepared 50 mM citrate buffer (pH, 4.0). Animals in control group received an equivalent volume of the citrate buffer. Seven days following the injection, blood samples were derived from a tail vein of the control and STZ treated animals. Development of the diabetic conditions in STZ treated animals was confirmed by blood glucose concentration measurements. Hyperglycemia (glucose levels of at least 300 mg/dl) was observed in all STZ treated rats, while glucose levels in all animals from the control group were normal (not more than 100 mg/dl).

Samples of normal rat serum (NRS) and diabetic rat serum (DRS) were obtained from five individual animals selected from control group and from five individual animals selected from STZ-treated group (day 7 after injection with STZ) using serum-separating tubes with clotting activator (Becton Dickinson, NJ,USA) according to the manufacturer's protocol.

A library of individual transcription reporter constructs, wherein each reporter contained a cis-regulatory element that was responsive to a particular TF, and each reporter RNA construct had a distinguishable reporter sequence, was constructed. The approach was used whereby each individual reporter construct is supplied with an identical reporter sequence that is supplied with a processing tag whose position varies within the library. The approach is described in detail in U.S. patent publication No. 2006/0160108.

According to this approach, transcripts of individual reporters can be distinguished by processing (i.e., digesting) of the corresponding RT-PCR products at the position of the processing tag (a unique Hpa I digest site) followed by separation of the pair of reporter sequence-specific primers, fluorescently labeled, processed (by digestion with the Hpa I restriction endonuclease), and resolved by using capillary electrophoresis. The relative activities of individual transcription reporter constructs were calculated as the values of corresponding individual peaks on the elctrophoregram and normalized on the mean value of all reporter peaks. Average values of each individual reporter construct obtained with five independent diabetic rat sera were compared with those obtained with five independent healthy rat sera.

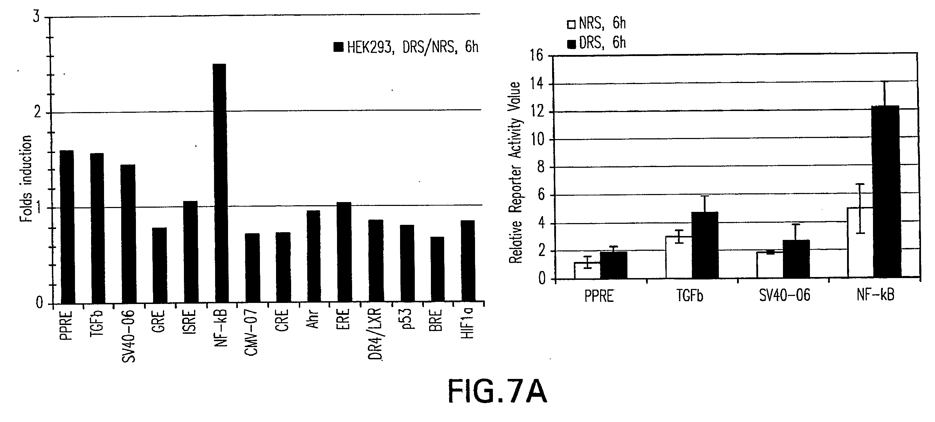

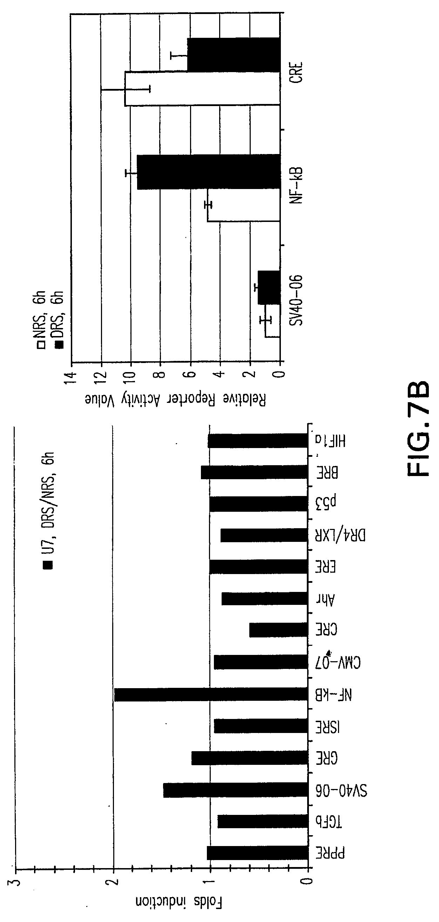

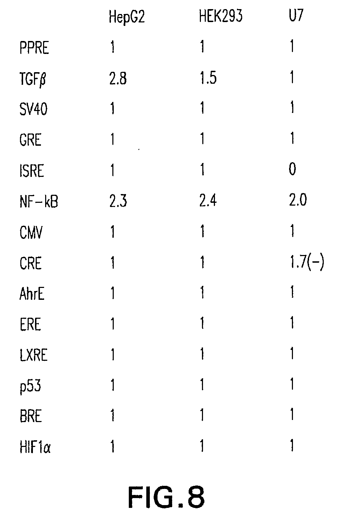

**Figures 7A** and **7B** show the profiles of induction/down-regulation of activities of individual reporters in, respectively, HEK293 and U7 biosensor cells treated with diabetic rat sera normalized to the activities of the reporters in reference cells treated with healthy rat sera. The profiles of transcriptional responses induced by diabetic sera at 6 hours in three different cell lines were diverse (compare **Figures 7A****,** **Figure 8A** and **Figure 8B**). NF-kB was the only transcription reporter construct, activity of which was consistently induced by diabetic serum in all biosensor cell lines. The degree of the NF-kB induction was also similar across all types of the biosensor cells: 2.3-fold induction in HepG2 cells, 2.4- fold induction in HEK293 cells and 2.0-fold induction in U7 cells. Increase of TGFβ reporter activity was limited to HepG2 (2.8-fold) and HEK293 (1.5-fold) biosensor cells. SV40 transcription was induced by diabetic serum in HEK293 (1.6-fold) and in U7 (1.8-fold) cells, but not in HepG2 cells. Noticeable down-regulation of CRE transcription was only observed in U7 cells (1.7-fold decrease). Thus, alterations of transcription reporter profiles induced by sera of diabetic animals are cell-type specific.

### 5.4. Example 4

This example demonstrates the assembly of data into a matrix form useful, for example, as a standard of comparison for diagnosing experimental diabetes in rats.

In examples 2 and 3, it was demonstrated that sera obtained from diabetic and normal animals can be distinguished on the basis of the differential alterations they exert in the profile of activities of transcription reporter constructs.

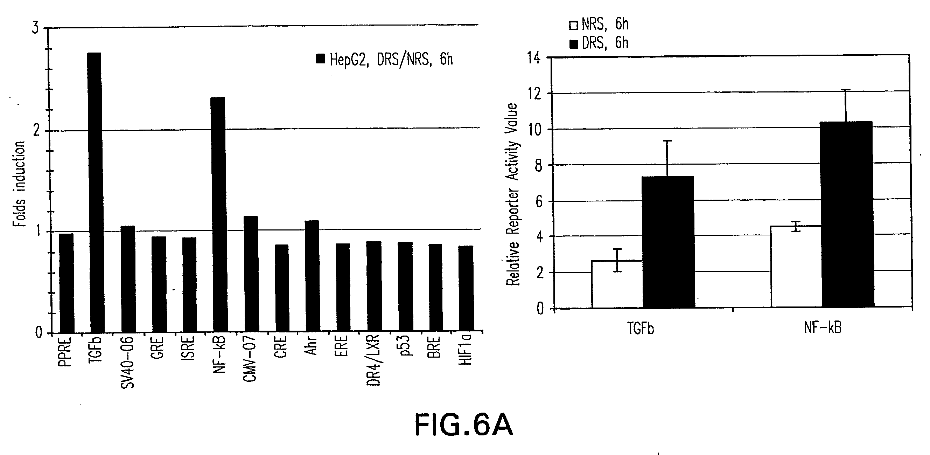

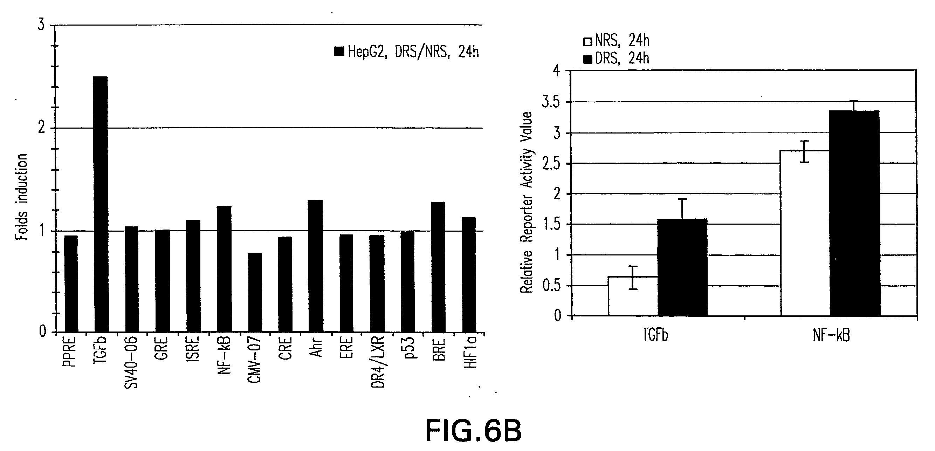

Transcriptional responses that are differentially induced by the sera extracted from diabetic animals in a variety of reporter cell types can be organized in a form of matrix, wherein each column represents profile of induction of library of transcription reporter constructs in individual reporter cell type, and, respectively, each raw represents profile of induction of individual transcription reporter construct across different reporter cell types. In this matrix, each pair of (reporter cell: reporter construct) is assigned a value that is equal to of fold-induction of average activity of given reporter construct in given reporter cell type by diabetic rat sera normalized to the average activity of given reporter construct in given reporter cell type treated by normal rat sera. If the average activity of given reporter construct in given reporter cell type treated with diabetic sera is not significantly different from the activity of given reporter construct in given reporter cell type treated with normal rat sera, the assigned value is equal 1. The significance of change in the activity of given reporter construct induced by diabetic sera may be assessed by using any standard statistical algorithm. Any one knowledgeable in the art will understand that result will also depend on number of individual diabetic and normal sera used and on variability of the responses exerted by different sera. For the purpose of the current example, the following criteria of significance of alteration of transcription reporter activity induced by diabetic sera were set: 1) the mean value of the activity in the presence of diabetic sera should be at least 1.5-fold different from that in the presence of normal sera; 2) spread of individual variations around the mean value (standard deviation) of activity measured in the presence of diabetic and normal sera should not overlap. Figure 8 illustrates an example of the prototypic matrix of significant transcriptional responses induced by diabetic sera in HepG2, HEK293 and U7 reporter cell lines, assembled based on the data shown in Figure 6 and Figure 7 (6 hours time points).

The prototypic matrix shown in Figure 8, can be easily extended by 1) expanding the library of individual transcription reporter units, and 2) by broadening the list of reporter cell types. Knowledgeable in the art will understand that composition and threshold of the significant alterations included in the matrix may change when size of the experiment (i.e. number of individual sera analyzed) is increased.

The matrix of transcription responses similar to that shown in Figure 7 provides a unique molecular signature of the STZ-induced diabetic condition. It can be used for the disease identification purposes.

## Claims

1. A method of determining biological activities of a biological sample which has been obtained from an organism, comprising:
(i) contacting the biological sample obtained from the organism with a cultured biosensor cell comprising a library of transcription reporter constructs for assessing transcriptional activities of multiple transcription factors (TFs) in the cultured biosensor cell,
wherein each transcription reporter construct in the library comprises a *cis*-regulatory element responsive to a TF and a reporter sequence,
wherein the reporter sequence in each transcription reporter construct in the library comprises a processing tag that is located in a position within the reporter sequence that differs from the positions of the processing tag in the reporter sequence of the other reporter constructs in the library, and
wherein the reporter sequences of the transcription reporter constructs, outside of the positions of the processing tags, are identical;
(ii) assessing relative levels of transcripts of the transcription reporter constructs in the library within the cultured biosensor cell to obtain a profile of activities of TFs regulating transcriptional activities of the reporter constructs in the library in the cultured biosensor cell;
(iii) comparing the profile of activities of TFs from the cultured biosensor cell contacted with the biological sample to a profile of activities of TFs in a cultured biosensor cell that was not contacted with the biological sample; and
(iv) generating a differential profile comprising differences between the profile of TF activities from the cultured biosensor cell contacted with the biological sample and the profile of activities of TFs in the cultured biosensor cell not contacted with the biological sample, wherein the differential profile represents the biological activities of the biological sample obtained from the organism.

2. The method of claim 1 further comprising
(v) contacting the biological sample obtained from the organism with a second cultured biosensor cell for a period of time different than the cell in step (i) was contacted, wherein the second cell comprises the library of transcription reporter constructs;
(vi) assessing relative levels of transcripts of the transcription reporter constructs in the library within each of the two cultured biosensor cells contacted in steps (i) and (v) to obtain a profile of activities of TFs regulating transcription of the transcription reporter constructs in the library in each of the two cultured biosensor cells;
(vii) comparing the profile of activities of the TFs in each of two cultured biosensor cells contacted with the biological sample with a profile of activities of the TFs in a cultured biosensor cell that was not contacted with the biological sample; and
(viii) generating a differential profile comprising differences in the profiles of the two biosensor cells contacted with the biological sample to a profile of activity of the TFs in cultured biosensor cell not contacted with the biological sample that represents the biological activities of the biological sample obtained from the organism.

3. The method of claim 1 or 2, wherein the profiles of activities of the TFs comprise activities of 5 TFs.

4. The method of claim 1, wherein the cultured biosensor cell is within a panel of cultured biosensor cells.

5. The method of claim 4, wherein the panel of cultured biosensor cells comprises 5 different types of cells.

6. The method of claim 4, wherein the panel of cultured biosensor cells includes fibroblasts, epithelial cells, immune cells, neural cells or stem cells.

7. The method of claim 4, wherein the panel of cultured biosensor cells is cultured in a tissue culture or organ culture.

8. The method of claim 1, 2 or 4, wherein the cultured biosensor cells are cultured in a homogenous cell culture or, a tissue culture.

9. The method of claim 1, 2 or 4, wherein said biological sample is saliva, blood, serum, cerebrospinal fluid, synovial fluid, urine, semen, breast milk, bile, tears, feces or an extract, concentrate or fractionate of saliva, blood, serum, cerebrospinal fluid, synovial fluid, urine, semen, breast milk, bile, tears or feces.

10. A method of identifying markers of a disease in a sample which has been obtained from an organism, comprising:
(i) determining, in accordance with the method of claim 1, biological activities of a biological sample obtained from the organism having the disease;
(ii) determining, in accordance with the method of claim 1, biological activities of a biological sample obtained from an organism not having the disease;
(iii) comparing the biological activities of the biological sample from the organism having the disease to the biological activities of the biological sample from the organism not having the disease; and
(iv) generating a profile of differences between the compared biological activities wherein the generated profile identifies markers of the disease.

11. The method of claim 10, wherein the organism with the disease and the organism not having the disease are human.

12. The method of claim 10, where the disease is an inflammatory disease, a metabolic disease, cancer, a neurodegenerative disease, a psychosomatic disease, or an infection.

## Patentansprüche

1. Verfahren zur Bestimmung biologischer Aktivitäten einer biologischen Probe, die aus einem Organismus erhalten worden ist, umfassend:
(i) in Kontakt bringen der aus dem Organismus erhaltenen biologischen Probe mit einer kultivierten Biosensor-Zelle umfassend eine Bibliothek von Transkriptionsreporterkonstrukten zur Bestimmung transkriptioneller Aktivitäten von einer Vielzahl von Transkriptionsfaktoren (TF's) in der kultivierten Biosensor-Zelle,
wobei jedes Transkriptionsreporterkonstrukt in der Bibliothek ein cis-regulatorisches Element, das auf einen TF und auf eine Reportersequenz reagiert, umfasst,
wobei die Reportersequenz in jedem Transkriptionsreporterkonstrukt in der Bibliothek eine Verarbeitungsmarkierung umfasst, die sich innerhalb der Reportersequenz an einer Position befindet, die sich von den Positionen der Verarbeitungsmarkierungen in den Reportersequenzen der anderen Reporterkonstrukte in der Bibliothek unterscheidet, und
wobei die Reportersequenzen der Transkriptionsreporterkonstrukte, abgesehen von den Positionen der Verarbeitungsmarkierungen, identisch sind;
(ii) Bestimmen der relativen Mengen von Transkripten der Transkriptionsreporterkonstrukte in der Bibliothek innerhalb der kultivierten Biosensor-Zelle, um ein Aktivitätsprofil von TF's, die die transkriptionellen Aktivitäten der Reporterkonstrukte in der Bibliothek in der kultivierten Biosensor-Zelle regeln, zu erhalten;
(iii) Vergleichen des Aktivitätsprofils von TF's aus der kultivierten Biosensor-Zelle, die mit der biologischen Probe in Kontakt gebracht worden ist, mit einem Aktivitätsprofil von TF's in einer kultivierten Biosensor-Zelle, die mit der biologischen Probe nicht in Berührung gebracht worden ist; und
(iv) Erzeugen eines Differentialprofils umfassend die Unterschiede zwischen dem Profil von TF-Aktivitäten aus der kultivierten Biosensor-Zelle, die mit der biologischen Probe in Kontakt gebracht worden ist und dem Aktivitätsprofil von TF's in der kultivierten Biosensor-Zelle, die mit der biologischen Probe nicht in Kontakt gebracht worden ist, wobei das Differentialprofil die biologischen Aktivitäten der biologischen Probe, die aus dem Organismus erhalten worden ist, darstellt.

2. Verfahren nach Anspruch 1, weiterhin umfassend
(v) in Kontakt bringen der biologischen Probe, die aus dem Organismus erhalten worden ist mit einer zweiten kultivierten Biosensor-Zelle für einen Zeitraum, der sich von dem Zeitraum, mit dem die Zelle in Schritt (i) in Kontakt gebracht worden ist, unterscheidet, wobei die zweite Zelle die Bibliothek von Transkriptionsreporterkonstrukten umfasst;
(vi) Bestimmen der relativen Mengen von Transkripten der Transkriptionsreporterkonstrukte in der Bibliothek innerhalb jeder der zwei kultivierten Biosensor-Zellen, die in den Schritten (i) und (v) in Kontakt gebracht worden sind, um ein Aktivitätsprofil von TF's, die die Transkription der Transkriptionsreporter-konstrukte in der Bibliothek in jeder der zwei kultivierten Biosensor-Zellen regeln, zu erhalten;
(vii) Vergleichen des Aktivitätsprofils der TF's in jeder der zwei kultivierten Biosensor-Zellen, die mit der biologischen Probe in Kontakt gebracht worden sind, mit einem Aktivitätsprofil der TF's in einer kultivierten Biosensor-Zelle, die mit der biologischen Probe nicht in Kontakt gebracht worden ist; und
(viii) Erzeugen eines Differentialprofils umfassend die Unterschiede zwischen den Profilen der zwei Biosensor-Zellen, die mit der biologischen Probe in Kontakt gebracht worden sind, und dem Aktivitätsprofil der TF's in der kultivierten Biosensor-Zelle, die mit der biologischen Probe nicht in Kontakt gebracht worden ist, was die biologischen Aktivitäten der biologischen Probe, die von dem Organismus erhalten worden ist, darstellt.

3. Verfahren nach Anspruch 1 oder 2, wobei die Aktivitätsprofile der TF's Aktivitäten von 5 TF's umfassen.

4. Verfahren nach Anspruch 1, wobei sich die kultivierte Biosensor-Zelle innerhalb eines Panels von Biosensor-Zellen befindet.

5. Verfahren nach Anspruch 4, wobei das Panel von kultivierten Biosensor-Zellen 5 verschiedene Zelltypen umfasst.

6. Verfahren nach Anspruch 4, wobei das Panel von kultivierten Biosensor-Zellen Fibroblasten, Epithelzellen, Immunzellen, Nervenzellen oder Stammzellen umfasst.

7. Verfahren nach Anspruch 4, wobei das Panel von kultivierten Biosensor-Zellen in einer Gewebekultur oder Organkultur kultiviert wird.

8. Verfahren nach Anspruch 1, 2 oder 4, wobei die kultivierten Biosensor-Zellen in einer homogenen Zellkultur oder Gewebekultur kultiviert werden.

9. Verfahren nach Anspruch 1, 2 oder 4, wobei die biologische Probe Speichel, Blut, Serum, Zerebrospinalflüssigkeit, Synovialflüssigkeit, Urin, Samen, Muttermilch, Gallenflüssigkeit, Tränen, Fäzes oder ein Extrakt, Konzentrat oder eine Fraktion von Speichel, Blut, Serum, Zerebrospinalflüssigkeit, Synovialflüssigkeit, Urin, Samen, Muttermilch, Gallenflüssigkeit, Tränen oder Fäzes ist.

10. Verfahren zur Identifizierung von Krankheitsmarkern in einer Probe, die aus einem Organismus erhalten worden ist, umfassend:
(i) Bestimmen, in Übereinstimmung mit dem Verfahren nach Anspruch 1, der biologischen Aktivitäten einer biologischen Probe, die aus dem Organismus mit der Krankheit erhalten worden ist;
(ii) Bestimmen, in Übereinstimmung mit dem Verfahren nach Anspruch 1, der biologischen Aktivitäten einer biologischen Probe, die aus dem Organismus ohne die Krankheit erhalten worden ist;
(iii) Vergleichen der biologischen Aktivitäten der biologischen Probe aus dem Organismus mit der Krankheit mit dem biologischen Aktivitäten der biologischen Probe aus dem Organismus ohne die Krankheit; und
(iv) Erzeugen eines Profils von Unterschieden zwischen den verglichenen biologischen Aktivitäten, wobei das erzeugte Profil die Krankheitsmarker aufdeckt.

11. Verfahren nach Anspruch 10, wobei der Organismus mit der Krankheit und der Organismus ohne die Krankheit Menschen sind.

12. Verfahren nach Anspruch 10, wobei die Krankheit eine Entzündungserkrankung, eine Stoffwechselerkrankung, Krebs, eine neurodegenerative Erkrankung, eine psychosomatische Erkrankung oder eine Infektion ist.

## Revendications

1. Procédé permettant de déterminer des activités biologiques d'un échantillon biologique qui a été obtenu d'un organisme, comprenant le fait de:
(i) mettre en contact l'échantillon biologique obtenu de l'organisme avec une cellule de biocapteur cultivée comprenant une librairie de constructions de rapporteurs de transcription pour évaluer les activités de transcription de multiples facteurs de transcription (FT) dans la cellule de biocapteur cultivée,
dans lequel chaque construction de rapporteurs de transcription dans la bibliothèque comprend un élément cis régulateur réagissant à une FT et une séquence de rapporteurs,
dans lequel la séquence de rapporteurs dans chaque construction de rapporteurs de transcription dans la bibliothèque comprend une étiquette de traitement qui se trouve en une position dans la séquence de rapporteurs qui diffère des positions de l'étiquette de traitement dans la séquence de rapporteurs des autres constructions de rapporteurs dans la bibliothèque, et
dans lequel les séquences de rapporteurs des constructions de rapporteurs de transcription, en dehors des positions des étiquettes de traitement, sont identiques;
(ii) évaluer les niveaux relatifs de transcription des constructions de rapporteurs de transcription dans la bibliothèque dans la cellule de biocapteur cultivée, pour obtenir un profil d'activités de FT régulant les activités de transcription des constructions de rapporteurs dans la bibliothèque dans la cellule de biocapteur cultivée;
(iii) comparer le profil d'activités de FT de la cellule de biocapteur cultivée mise en contact avec l'échantillon biologique avec un profil d'activités de FT dans une cellule de biocapteur cultivée qui n'a pas été mis en contact avec l'échantillon biologique; et
(iv) générer un profil différentiel comprenant des différences entre le profil d'activités de FT de la cellule de biocapteur cultivée mise en contact avec l'échantillon biologique et le profil d'activités de FT dans la cellule de biocapteur cultivée qui n'est pas mise en contact avec l'échantillon biologique, où le profil différentiel représente les activités biologiques de l'échantillon biologique obtenu de l'organisme.

2. Procédé selon la revendication 1, comprenant par ailleurs le fait de
(v) mettre en contact l'échantillon biologique obtenu de l'organisme avec une deuxième cellule de biocapteur cultivée pendant un laps de temps différent de celui pendant lequel la cellule à l'étape (i) était en contact, où la deuxième cellule comprend la bibliothèque de constructions de rapporteurs de transcription;
(vi) évaluer les niveaux relatifs de transcription des constructions de rapporteurs de transcription dans la bibliothèque dans chacune des deux cellules de biocapteur cultivées mises en contact aux étapes (i) et (v), pour obtenir un profil d'activités de FT régulant la transcription des constructions de rapporteurs de transcription dans la bibliothèque dans chacune des deux cellules de biocapteurs cultivées;
(vii) comparer le profil d'activités des FT dans chacune des deux cellules de biocapteur cultivées mises en contact avec l'échantillon biologique avec un profil d'activités des FT dans une cellule de biocapteur cultivée qui n'a pas été mise en contact avec l'échantillon biologique; et
(viii) générer un profil différentiel comprenant des différences entre les profils des deux cellules de biocapteur mises en contact avec l'échantillon biologique et un profil d'activités des FT dans la cellule de biocapteur cultivée qui n'a pas été mise en contact avec l'échantillon biologique qui représente les activités biologiques de l'échantillon biologique obtenu de l'organisme.

3. Procédé selon la revendication 1 ou 2, dans lequel les profils d'activités des FT comprennent des activités de 5 FT.

4. Procédé selon la revendication 1, dans lequel la cellule de biocapteur cultivée se trouve dans un panneau de cellules de biocapteur cultivées.

5. Procédé selon la revendication 4, dans lequel le panneau de cellules de biocapteur cultivées comprend 5 types de cellules différents.

6. Procédé selon la revendication 4, dans lequel le panneau de cellules de biocapteur cultivées comporte des fibroblastes, des cellules épithéliales, des cellules immunitaires, des cellules neuronales ou des cellules souches.

7. Procédé selon la revendication 4, dans lequel le panneau de cellules de biocapteur cultivées est cultivé dans une culture de tissu ou une culture d'organe.

8. Procédé selon la revendication 1, 2 ou 4, dans lequel les cellules de biocapteur cultivées sont cultivées dans une culture cellulaire homogène ou une culture tissulaire.

9. Procédé selon la revendication 1, 2 ou 4, dans lequel ledit échantillon biologique est de la salive, du sang, du sérum, du liquide céphalo-rachidien, du liquide synovial, de l'urine, du sperme, du lait maternel, de la bile, des larmes, des matières fécales ou un extrait, un concentré ou un fractionnement de salive, de sang, de sérum, de liquide céphalo-rachidien, de liquide synovial, d'urine, de sperme, de lait maternel, de bile, de larmes ou de matières fécales.

10. Procédé permettant d'identifier des marqueurs d'une maladie dans un échantillon qui a été obtenu d'un organisme, comprenant le fait de:
(i) déterminer, selon le procédé de la revendication 1, des activités biologiques d'un échantillon biologique obtenu de l'organisme présentant la maladie;
(ii) déterminer, selon le procédé de la revendication 1, des activités biologiques d'un échantillon biologique obtenu d'un organisme ne présentant pas la maladie;
(iii) comparer les activités biologiques de l'échantillon biologique de l'organisme présentant la maladie avec les activités biologiques de l'échantillon biologique de l'organisme ne présentant pas la maladie; et
(iv) générer un profil de différences entre les activités biologiques comparées, où le profil généré identifie des marqueurs de la maladie.

11. Procédé selon la revendication 10, dans lequel l'organisme présentant la maladie et l'organisme ne présentant pas la maladie sont humains.

12. Procédé selon la revendication 10, dans lequel la maladie est une maladie inflammatoire, une maladie métabolique, le cancer, une maladie neurodégénérative, une maladie psychosomatique, ou une infection.
